# EUROPÄISCHE PATENTANMELDUNG

(11) **EP 3 062 249 A1**
(43) Veröffentlichungstag der Anmeldung: **31.08.2016**
(21) Anmeldenummer: 16153681.8
(22) Anmeldetag: 01.02.2016
(51) Int. Cl.: G06F 19/00, H04R 25/00, H04R 29/00

(54) **VERFAHREN ZUR ERMITTLUNG VON TRÄGERSPEZIFISCHEN NUTZUNGSDATEN EINES HÖRGERÄTS, VERFAHREN ZUR ANPASSUNG VON HÖRGERÄTEEINSTELLUNGEN EINES HÖRGERÄTS, HÖRGERÄTESYSTEM UND EINSTELLEINHEIT FÜR EIN HÖRGERÄTESYSTEM**

(30) Priorität: 24.02.2015 DE 102015203288
(71) Anmelder: Sivantos Pte. Ltd., Singapore 139959 (SG)
(72) Erfinder: REINLEIN, Claus, 90559 Burgthann (DE)
(74) Vertreter: FDST Patentanwälte

(57) **Zusammenfassung**

Erfindungsgemäß wird mittels einer einem Hörgerät (2) zugeordneten, von diesem separaten und zur unidirektionalen Signalübertragung auf das Hörgerät (2) eingerichteten Analyseeinheit (4) ein trägerspezifisches Problem mit dem aktuellen Betriebszustand ermittelt und in der Analyseeinheit (4) als erster Teildatensatz (TSᵢ) gespeichert. Daraufhin wird von der Analyseeinheit (4) ein Steuersignal (19) an das Hörgerät (2) ausgegeben, wobei auf dessen Empfang hin das Hörgerät (2) eine Anzahl von Betriebsdaten als zweiten Teildatensatz (THᵢ) speichert. Diese beiden Teildatensätze (TSᵢ, THᵢ) werden später mittels einer Einstelleinheit (5) aus der Analyseeinheit (4) und dem Hörgerät (2) abgerufen, in Verbindung gesetzt, ausgewertet und daraufhin neue Hörgeräteeinstellungen für das Hörgerät (2) ermittelt.

## Beschreibung

Die Erfindung bezieht sich auf ein Verfahren zur Ermittlung von trägerspezifischen Nutzungsdaten eines Hörgeräts sowie auf ein Verfahren zur Anpassung von Hörgeräteeinstellungen. Des Weiteren bezieht sich die Erfindung auf ein Hörgerätesystem sowie auf eine Einstelleinheit für ein solches Hörgerätesystem.

Hörgeräte dienen herkömmlicherweise Personen mit eingeschränktem Hörvermögen dazu, die Verminderung ihres Hörvermögens zumindest teilweise auszugleichen. Hierfür umfassen Hörgeräte meist ein Mikrofon (gegebenenfalls auch mehrere Mikrofone) zur Aufnahme von Geräuschen aus der Umgebung (d.h. insbesondere Sprache und Musik sowie sonstige Tonsignale) und einen Lautsprecher (häufig auch als "Hörer" bezeichnet) zur Weitergabe der aufgenommenen Umgebungsgeräusche an ein Ohr des Hörgeräteträgers. Alternativ zu dem Lautsprecher können die Hörgeräte auch andere Übertragungsvorrichtungen auf das Ohr oder das Hörzentrum des Hörgeräteträgers aufweisen (beispielsweise spezielle Implantate zur Schallübertragung auf einen Schädelknochen oder ein sogenanntes Cochlea-Implantat). Zur Verstärkung und/oder anderweitigen Verarbeitung der über das Mikrofon (bzw. die Mikrofone) aufgenommenen Tonsignale umfassen herkömmliche Hörgeräte eine zwischen das Mikrofon und den Lautsprecher geschaltete Steuereinheit.

Diese Steuereinheit ist regelmäßig dazu eingerichtet, die aufgenommenen Tonsignale frequenzabhängig unterschiedlich zu verstärken, sodass beispielsweise Tonsignale in den Frequenzbändern, in denen der Schall menschlicher Sprache liegt, mehr verstärkt werden als andere Tonsignale, wie beispielsweise Umgebungslärm. Da jeder Hörgeräteträger eine spezifische, gegebenenfalls frequenzabhängige Beeinträchtigung seines Hörvermögens aufweist, ist die Steuereinheit der Hörgeräte insbesondere auch dazu eingerichtet, im Hinblick auf die Wiedergabecharakteristik der aufgenommenen Tonsignale individuell an den jeweiligen Hörgeräteträger bzw. dessen Hörvermögen angepasst zu werden. Beispielsweise werden bei dieser Anpassung die Werte für eine frequenzabhängige Verstärkung und/oder Dämpfung trägerspezifisch voreingestellt.

Des Weiteren umfasst die Steuereinheit meist auch einen als Klassifikator bezeichneten Baustein, der dazu eingerichtet und vorgesehen ist, anhand der Charakteristik der aufgenommenen Geräusche (d. h. der aufgenommenen Tonsignale) eine Hörsituation zu erkennen. Bei einer solchen Hörsituation handelt es sich beispielsweise um ein Gespräch in Ruhe zwischen dem Hörgeräteträger und einer zweiten Person oder um Fernsehen. Bei diesen beiden Hörsituationen liegt üblicherweise nur ein geringer Anteil an (unerwünschten) Nebengeräuschen (auch als Störsignale bezeichnet) vor. Andere Hörsituationen können beispielsweise für ein Gespräch bei Umgebungslärm, den Aufenthalt des Hörgeräteträgers an einen öffentlichen Platz, im Straßenverkehr, in der Natur oder dergleichen (jeweils mit vergleichsweise vielen Störgeräuschen) stehen. In einer der Steuereinheit des Hörgeräts zugeordneten Speichereinheit sind dabei häufig verschiedene Programme hinterlegt, bei deren Ausführung die aufgenommenen Tonsignale abhängig von der jeweiligen Hörsituation unterschiedlich verstärkt, gedämpft (und gegebenenfalls ausgeblendet) werden und somit eine an die jeweilige Hörsituation angepasste Ausgabe über den Lautsprecher des Hörgeräts erfolgt. Die Steuereinheit ist hierbei meist dazu eingerichtet, das jeweilige Programm in Abhängigkeit von der von dem Klassifikator erkannten Hörsituation selbständig (automatisch) auszuwählen und die Signalverarbeitung entsprechend durchzuführen. Eine manuelle Einstellung der Programme ist dabei aber häufig (zusätzlich) auch möglich.

Die Anpassung der Einstellung der (Hörgeräte-)Steuereinheit auf das Hörvermögen des Hörgeräteträgers wird herkömmlicherweise von speziell geschultem Personal, insbesondere von einem Hörgeräteakustiker, vorgenommen. Dabei werden die jeweiligen Einstellungen (Parameter) auch für jedes (Betriebs-)Programm separat (gegebenenfalls unter Zuhilfenahme von Erfahrungswerten) angepasst.

Jedoch kann auch der Hörgeräteakustiker nicht jede Hörsituation (vorausschauend) abbilden und dem Hörgeräteträger eine entsprechende Testumgebung zum Testen der jeweiligen Klangcharakteristik des Hörgeräts bieten. Bei der ersten Anpassung des Hörgeräts (bzw. dessen Steuereinheit) handelt es sich folglich regelmäßig vielmehr nur um eine erste, grobe Annäherung an die für den jeweiligen Hörgeräteträger für ein subjektiv komfortables Hörempfinden erforderlichen Hörgeräteeinstellungen. Probleme mit dem Hörgerät - d.h. insbesondere Wünsche nach einer je nach Hörsituation anderen Klangcharakteristik des Hörgeräts - erkennt der Hörgeräteträger meist erst dann, wenn bei der Nutzung des Hörgeräts eine entsprechende spezifische Hörsituation auch tatsächlich vorliegt. Eine genauere Anpassung des Hörgeräts an die zum Zeitpunkt eines solchen Problems vorliegende Hörsituation erfolgt dann herkömmlicherweise zu einem späteren Zeitpunkt bei dem Hörgeräteakustiker.

Der Erfindung liegt die Aufgabe zugrunde, die Anpassung von Hörgeräteeinstellungen zu verbessern.

Diese Aufgabe wird erfindungsgemäß gelöst durch ein Verfahren zur Ermittlung von trägerspezifischen Nutzungsdaten eines Hörgeräts mit den Merkmalen des Anspruchs 1. Des Weiteren wird die Aufgabe erfindungsgemäß gelöst durch ein Verfahren zur Anpassung von Hörgeräteeinstellungen mit den Merkmalen des Anspruchs 10. Weiterhin wird die Aufgabe außerdem erfindungsgemäß gelöst durch ein Hörgerätesystem mit den Merkmalen des Anspruchs 11. Ebenfalls wird die Aufgabe erfindungsgemäß gelöst durch eine Einstelleinheit für ein Hörgerätesystem mit den Merkmalen des Anspruchs 12. Vorteilhafte und teils für sich erfinderische Ausführungsformen und Weiterentwicklungen der Erfindung sind in den Unteransprüchen und der nachfolgenden Beschreibung dargelegt.

Das erfindungsgemäße Verfahren dient zur Ermittlung von trägerspezifischen Nutzungsdaten eines Hörgeräts und wird vorzugsweise mittels eines Hörgerätesystems (vorzugsweise gebildet durch ein Hörgerät und eine diesem Hörgerät spezifisch zugeordnete und von dem Hörgerät separate Analyseeinheit) durchgeführt. Die Analyseeinheit ist dabei nur zur unidirektionalen Signalübertragung auf das Hörgerät eingerichtet und vorgesehen (d.h. die Analyseeinheit kann keine Informationen von dem Hörgerät empfangen). Unter dem Begriff Hörgerät wird hier und im Folgenden sowohl ein einzelnes (für ein Ohr vorgesehenes) Hörgerät als auch ein Hörgerätepaar, das für beide Ohren eines Hörgeräteträgers (zur binauralen Versorgung) vorgesehen ist, verstanden.

Verfahrensgemäß wird mittels der dem Hörgerät spezifisch zugeordneten Analyseeinheit ein trägerspezifisches Problem des Hörgeräteträges mit dem aktuellen Betriebszustand des Hörgeräts ermittelt. Das ermittelte trägerspezifische Problem wird daraufhin in einer der Analyseeinheit zugeordneten Speichereinheit als Teil eines ersten Teildatensatzes der zu ermittelnden Betriebsdaten hinterlegt. Anschließend wird von der Analyseeinheit ein Steuersignal vorzugsweise drahtlos an das Hörgerät ausgegeben. Auf den Empfang des Steuersignals in dem Hörgerät wird eine Anzahl von vorzugsweise hörgerätespezifischen Betriebsdaten des aktuellen Betriebszustands des Hörgeräts ermittelt und als zweiter Teildatensatz der zu ermittelnden Nutzungsdaten in einer Speichereinheit des Hörgeräts hinterlegt. Sowohl der erste Teildatensatz als auch der zweite Teildatensatz werden dabei derart in der jeweiligen Speichereinheit hinterlegt, dass die beiden Teildatensätze auch nach einer Veränderung des aktuellen Betriebszustands des Hörgeräts in der jeweiligen Speichereinheit (auslesbar) hinterlegt bleiben.

Bei den trägerspezifischen Nutzungsdaten des Hörgeräts handelt es sich somit einerseits um ein Problem des Hörgeräteträgers mit dem Hörgerät. Beispielsweise ist dem Hörgeräteträger der wiedergegebene Umgebungslärm zu laut oder er hört seine eigene Stimme zu laut, zu leise, mit Nachhall oder dergleichen. Andererseits umfassen die trägerspezifischen Nutzungsdaten auch aktuelle Betriebsdaten des Hörgeräts. Bei den Betriebsdaten handelt es sich insbesondere um Eingangspegel unterschiedlicher Frequenzbänder (d. h. die Umgebungslautstärke), ein aktuell "laufendes" (d. h. ausgeführtes) Programm des Hörgeräts, (programmabhängige) Einstellwerte (oder auch: Parameter) für Frequenzkompression, für frequenzabhängige Verstärkung und/oder Dämpfung, eine von einem gegebenenfalls in dem Hörgerät vorhandenen Klassifikator ausgegebene Klassifikation der aktuellen Hörsituation, ein aktuell vom Hörgeräteträger eingestellter Wert der (Ausgabe-)Lautstärke, (bei einem Hörgerätepaar) die Balance zwischen linkem und rechtem Hörgerät und dergleichen.

Bei der Analyseeinheit handelt es sich vorzugsweise um eine zur Ermittlung des trägerspezifischen Problems und zur Ausgabe des Steuersignals eingerichtete Softwareapplikation, die bevorzugt auf einem Smartphone oder alternativ auf einer dem Hörgerät zugeordneten Fernbedienung ausführbar installiert ist. Um zu verhindern, dass "fremde" Hörgeräte, d.h. Hörgeräte von sich in der Nähe befindenden anderen Hörgeräteträgern, auf das Steuersignal der Analyseeinheit reagieren, wird die Analyseeinheit in zweckmäßiger Ausführung vor dem ersten Senden des Steuersignals in einem als "Pairing" bezeichneten und vorzugsweise von dem erfindungsgemäßen Verfahren unabhängigen Verfahrensschritt eindeutig auf das betreffende Hörgerät abgestimmt (diesem somit zugeordnet). Dadurch wird erreicht, dass insbesondere durch Übermittlung einer hörgerätespezifischen Adresse in dem Steuersignal nur das zugeordnete (adressierte) Hörgerät das Steuersignal als solches erkennt. Dadurch, dass die beiden gespeicherten Teildatensätze von einer Veränderung des aktuellen Betriebszustandes des Hörgeräts, wie beispielsweise der Aktivierung eines anderen Programms oder einer Veränderung der Lautstärke etc., unbeeinflusst hinterlegt bleiben, können beide Teildatensätze vorteilhafterweise zu einem späteren Zeitpunkt aus der jeweiligen Speichereinheit der Analyseeinheit und des Hörgeräts abgerufen und zur (Nach-)Anpassung der Hörgeräteeinstellungen - insbesondere durch einen Hörgeräteakustiker - herangezogen werden. Durch die Speicherung (und insbesondere den späteren Abruf) der beiden Teildatensätze kann die zu dem Zeitpunkt, zu dem das Problem auftrat und mittels der Analyseeinheit ermittelt wurde, vorliegende Hörsituation (zu einem späteren Zeitpunkt) auf einfache Weise präzise nachgebildet und die für das Problem kausalen Parameter besonders präzise identifiziert werden. Es wird also vorteilhafterweise der Nachteil überwunden, dass sich Hörgeräteträger - wenn sie zur Nachanpassung ihren Hörgeräteakustiker aufsuchen - häufig nicht mehr ausreichend präzise an die zum Zeitpunkt des Problems vorliegende Hörsituation erinnern können.

Grundsätzlich ist es im Rahmen der Erfindung denkbar, dass das Steuersignal mittels eines magnetisch oder induktiv basierten oder eines funkbasierten (z. B. WLAN oder Bluetooth) Kommunikationsverfahrens auf das Hörgerät übertragen wird. Vorzugsweise ist die Analyseeinheit (insbesondere alle für die Steuerung des Hörgeräts auf dem Smartphone oder der Fernbedienung implementierten Softwareapplikationen) allerdings dazu eingerichtet, die Daten und Steuerbefehle akustisch an das Hörgerät zu übertragen. In diesem Fall wird also in zweckmäßiger Ausführung das Steuersignal mittels eines der Analyseeinheit zugeordneten Lautsprechers akustisch (d. h. als Tonsignal) an das Hörgerät ausgegeben. Vorzugsweise wird hierbei für das Steuersignal eine Übertragungsfrequenz mit einer Bandbreite zwischen etwa 12 und 16 Kilohertz, insbesondere um etwa 15 Kilohertz herangezogen.

In einer zweckmäßigen Ausführung des Verfahrens werden zusätzlich zu dem trägerspezifischen Problem mittels wenigstens eines der Analyseeinheit zugeordneten Sensors weitere für die aktuelle Situation des Hörgeräteträgers charakteristische Daten (im Folgenden als "Situationsdaten" bezeichnet) erfasst und als weiterer Teil des ersten Teildatensatzes hinterlegt. Vorzugsweise wird hierbei als Sensor ein (bei einem Smartphone ohnehin vorhandenes) Mikrofon herangezogen. Mittels dieses Mikrofons werden als Situationsdaten insbesondere akustische Informationen wie die (aktuelle) Umgebungslautstärke (in Form von frequenzabhängigen Pegeln) und gegebenenfalls Frequenzschwankungen erfasst. Diese (akustischen) Situationsdaten können wiederum zweckmäßigerweise bei einer späteren Auswertung der ersten und zweiten Teildatensätze dazu genutzt werden, die Funktion des gegebenenfalls im Hörgerät vorhandenen Klassifikators zu verifizieren. Zusätzlich werden als weitere Situationsdaten beispielsweise auch das aktuelle Datum und die Uhrzeit ermittelt. Hieraus kann bei der späteren Auswertung der Teildatensätze auf ein gegebenenfalls tageszeitabhängiges Problem geschlossen werden. Als weiterer Sensor kann im Rahmen der Erfindung beispielsweise auch ein GPS-Sensor (der in einem Smartphone meist ebenfalls vorhanden ist) zur Ermittlung von Standortdaten (als Situationsdaten) herangezogen werden. Grundsätzlich ist es im Rahmen der Erfindung insbesondere auch denkbar, dass die mittels des bzw. der zusätzlichen Sensoren ermittelten Situationsdaten (nach dem Auslesen aus der Analyseeinheit) dem Hersteller des Hörgeräts zur Verfügung gestellt werden, so dass dieser die Situationsdaten bei der Entwicklung und (werksseitigen) Voreinstellung von Hörgeräten nutzen kann.

Um zwischen dem Auftreten eines "ersten" Problems, dessen vorstehend beschriebener Ermittlung sowie Hinterlegung der beiden Teildatensätze in den jeweiligen Speichereinheiten, und der Nachanpassung der Hörgeräteeinstellungen (beim Hörgeräteakustiker) gegebenenfalls weitere auftretende Probleme ermitteln und jeweils (einen zugeordneten ersten und zweiten Teildatensatz) speichern zu können, wird in einer besonders zweckmäßigen Ausführung des Verfahrens sowohl dem ersten Teildatensatz als auch dem zweiten Teildatensatz jeweils ein Marker zugewiesen. Diese beiden Marker dienen einer eindeutigen (insbesondere zeitlichen) Zuordnung der beiden Teildatensätze zueinander - d. h. des mittels der Analyseeinheit ermittelten Problems und der diesbezüglich in dem Hörgerät gespeicherten Betriebsdaten. Beispielsweise wird als Marker dem jeweiligen Teildatensatz ein (gemeinsamer) Zeitstempel (d. h. Datum und Uhrzeit) zugeordnet. Vorzugsweise handelt es sich bei dem Marker allerdings um einen für den ersten und für den zweiten Teildatensatz gleichen Index, insbesondere einen fortlaufend erhöhten Zähler, mittels dessen eine eindeutige Zuordnung der beiden Teildatensätze zueinander bei besonders geringem Speicherbedarf (sowie geringem Datenübertragungsvolumen) ermöglicht wird.

In bevorzugter Ausführung wird der Marker für den zweiten Teildatensatz oder zumindest ein Befehl zur Generierung dieses Markers in einer Steuereinheit des Hörgeräts mittels des Steuersignals von der Analyseeinheit auf das Hörgerät übertragen. Beispielsweise wird als Marker unmittelbar ein Zählindex von 1 bis 20 oder bis 50 (je nach Größe der Speichereinheit und somit vorgehaltenen Speicherplätzen für die Teildatensätze) übertragen. In einer alternativen Ausführung wird, insbesondere um die mittels des Steuersignals zu übertragenden Informationen möglichst gering zu halten, als Befehl zur Generierung des Markers lediglich ein einstelliger Zähler übertragen, dessen Wert von der Steuereinheit als Index herangezogen wird. Bei erneutem Empfang eines bereits vergebenen Index wird eine führende Zehnerstelle für den Index eingeführt und/oder gegebenenfalls um 1 erhöht. Mit anderen Worten sendet die Analyseeinheit zur Generierung des Markers nur Ziffern von 0 bis 9 und die Steuereinheit des Hörgeräts erzeugt zunächst die Indizes 0 bis 9 und anschließend bei erneutem Empfang beispielsweise der Ziffer 1 den Index 11, 21 usw..

In zweckmäßiger Ausführung des Verfahrens wird dem Hörgeräteträger zur Ermittlung des trägerspezifischen Problems von der Analyseeinheit im Rahmen einer Frage-Antwort-Routine eine Anzahl von Fragen zur Beantwortung vorgegeben. Diese Fragen werden dabei insbesondere einem in (der Speichereinheit) der Analyseeinheit hinterlegten Fragenkatalog entnommen. Zur Beantwortung werden dem Hörgeräteträger dabei vorzugsweise Antworten (nach einem "multiple choice"-Prinzip oder als Falschoder Richtigauswahl) vorgegeben. Die Frage-Antwort-Routine wird dabei (in bekannter Weise) derart abgearbeitet, dass in Abhängigkeit von der gewählten Antwort eine weitere (in Richtung auf eine gegenüber dem vorherigen Schritt verringerte Auswahl von Problemen hinführende) Frage aus dem Fragenkatalog ausgewählt und dem Hörgeräteträger vorgelegt wird. Der Fragenkatalog ist dabei zweckmäßigerweise auf eine insbesondere auf Erfahrungswerten basierende Anzahl und Auswahl von möglichen, d. h. herkömmlicherweise auftretenden Problemen von Hörgeräteträgern mit dem Betriebszustand ihres Hörgeräts abgestimmt. Bei Durchführung der Frage-Antwort-Routine wird also zweckmäßigerweise je nach Beantwortung der Fragen die Anzahl der möglicherweise vorliegenden Probleme von der Analyseeinheit immer weiter eingeschränkt, bis ein konkretes Problem ermittelt ist. Vorzugsweise wird nach einer abschließenden Frage das von der Analyseeinheit ermittelte Problem dem Hörgeräteträger zur Bestätigung vorgelegt und erst auf Bestätigung des Hörgeräteträgers hin der erste Teildatensatz gespeichert und das Steuersignal ausgegeben.

Um eine besonders hohe Nutzerfreundlichkeit zu erreichen, bietet die Analyseeinheit in zweckmäßiger Ausführung - insbesondere für den Fall, dass mittels der Frage-Antwort-Routine das vorliegende trägerspezifische Problem nicht ermittelt werden konnte - dem Hörgeräteträger zusätzlich (zur Bestätigung des ermittelten Problems) eine direkte Eingabe einer Beschreibung des von dem Hörgeräteträger wahrgenommenen Problems an. Insbesondere für den Fall, dass die Analyseeinheit als Softwareapplikation auf einem Smartphone implementiert ist, öffnet die Analyseeinheit dazu ein Texteingabefenster oder bietet eine Spracheingabe des Problems über das Mikrofon des Smartphones an. Dadurch kann auf einfache Weise auch ein bisher nicht bekanntes (oder nicht hinterlegtes) Problem erfasst werden.

Im Rahmen der Erfindung ist es grundsätzlich denkbar, dass die Frage-Antwort-Routine in vorgegebenen Zeitabständen und/oder nach einer Änderung des aktuellen Programms automatisch startet. In bevorzugter Ausführung wird die Frage-Antwort-Routine jedoch erst auf einen Befehl (d. h. auf eine entsprechende Eingabe des Hörgeräteträgers in der Analyseeinheit) hin gestartet.

Grundsätzlich ist es im Rahmen der Erfindung möglich, dass als zweiter Teildatensatz in dem Hörgerät alle vorhandenen Betriebsdaten gespeichert werden. Um jedoch den je (zweitem) Teildatensatz erforderlichen Speicherbedarf möglichst gering zu halten, wird für den zweiten Teildatensatz jedoch nur eine (vorzugsweise fest) vorgegebene und gegenüber allen im Hörgerät vorhandenen Betriebsdaten eingeschränkte (d. h. verringerte) Anzahl der Betriebsdaten herangezogen. Bei diesen vorgegebenen Betriebsdaten handelt es sich insbesondere um die vorstehend beschriebenen. Somit werden vorzugsweise für jeden zweiten Teildatensatz stets die Werte der gleichen Betriebsdaten in der Speichereinheit des Hörgeräts hinterlegt. Zweckmäßigerweise sind die als zweiter Teildatensatz zu speichernden Betriebsdaten auf die (insbesondere auch dem Fragenkatalog der Analyseeinheit zugrunde gelegten) herkömmlichen Probleme von Hörgeräteträgern mit ihren jeweiligen Hörgeräten abgestimmt. Das heißt, dass es sich bei den zu speichernden Betriebsdaten um die für diese herkömmlichen Probleme kausalen und somit zur Behebung dieser Probleme erforderlichen, relevanten Betriebsdaten handelt.

In alternativer Ausführung wird in Abhängigkeit von dem jeweils ermittelten Problem aus allen im Hörgerät vorhandenen Betriebsdaten eine (gegenüber der Gesamtzahl) eingeschränkte Anzahl der Betriebsdaten ausgewählt und diese (problemspezifisch) für den zweiten Teildatensatz herangezogen. Im Rahmen der Erfindung ist es dabei denkbar, dass die Auswahl der zu speichernden Betriebsdaten bereits im Analysegerät getroffen wird, oder dass alternativ das ermittelte Problem (als Informationsinhalt des Steuersignals) an das Hörgerät übermittelt und die jeweilige Auswahl der zu speicherenden Betriebsdaten von der Steuereinheit des Hörgeräts getroffen wird.

Bei dem erfindungsgemäßen Verfahren zur Anpassung von (Hörgeräte-)Einstellungen des Hörgeräts, insbesondere zur Nachanpassung ("Nachjustierung") der Einstellungen (Parameter) des Hörgeräts, wird dem vorstehend beschriebenen Verfahren zur Ermittlung der Nutzungsdaten ein weiterer Verfahrensschritt hinzugefügt. Dabei wird mittels einer zur bidirektionalen Kommunikation mit dem Hörgerät und mit der Analyseeinheit eingerichteten Einstelleinheit ein trägerspezifischer Nutzungsdatensatz des Hörgeräts erfasst. Bei diesem Nutzungsdatensatz handelt es sich um den ersten Teildatensatz und den zugehörigen zweiten Teildatensatz. Diese beiden Teildatensätze werden mittels der Einstelleinheit jeweils aus der Analyseeinheit und aus dem Hörgerät (insbesondere aus den jeweiligen Speichereinheiten) ausgelesen. Zur Bildung des Nutzungsdatensatzes wird anschließend der erste Teildatensatz mit dem zugehörigen zweiten Teildatensatz - vorzugsweise anhand ihrer zugeordneten Marker - in Verbindung gesetzt. In einem nachfolgenden Schritt werden beide Teildatensätze gemeinsam ausgewertet. Anhand des Ergebnisses der Auswertung werden (insbesondere von der Einstelleinheit automatisch) neue Einstellungen für das Hörgerät ermittelt und vorzugsweise auf eine Bestätigung hin (die insbesondere durch den Hörgeräteakustiker erfolgt) an das Hörgerät übertragen.

Durch die Speicherung der (problembezogenen) Nutzungsdaten und deren späteren Abruf mittels der Einstelleinheit wird eine einfache und präzise Ermittlung und Anpassung der aktuellen Parameter des Hörgeräts, also der vorliegenden Hörgeräteeinstellungen ermöglicht. Insbesondere kann der Hörgeräteakustiker und/oder die Einstelleinheit anhand der im Hörgerät hinterlegten Betriebsdaten die zum Zeitpunkt der Problemerkennung vorliegende Hörsituation besonders präzise nachbilden, ohne auf das meist vergleichsweise unpräzise Erinnerungsvermögen des Hörgeräteträgers angewiesen zu sein.

Die bidirektionale Kommunikation zwischen der Einstelleinheit und dem Hörgerät bzw. der Analyseeinheit erfolgt dabei vorzugsweise kabelgebunden. Alternativ ist im Rahmen der Erfindung auch jeweils eine drahtlose, insbesondere funkbasierte Kommunikation möglich.

Für den Fall, dass das Hörgerät einen Klassifikator zur Ermittlung ("Klassifikation") der aktuellen Hörsituation umfasst und die Analyseeinheit in dem ersten Teildatensatz auch Situationsdaten über die Umgebungslautstärke (sowie ggf. Frequenzschwankungen) speichert, wird mittels der Einstelleinheit in einer zweckmäßigen Ausführung aus den von der Analyseeinheit gespeicherten, akustischen Situationsdaten eine (Vergleichs-)Hörsituation bestimmt und diese mit der zum Zeitpunkt des Problems mittels des Klassifikators ermittelten (hörgerätespezifischen) Hörsituation verglichen. Vorzugsweise wird bei gegebenenfalls voneinander abweichenden Hörsituationen eine Warnung ausgegeben. Im Rahmen der Erfindung ist es dabei denkbar, dass auf diese Warnung hin die Einstelleinheit automatisch neue Einstellungen für den Klassifikator ermittelt und (vorzugsweise dem Hörgerätetechniker) zur Anpassung des Hörgeräts vorschlägt. Alternativ, vorzugsweise aber zumindest vor einer Bestätigung der Übertragung der neuen Einstellungen für den Klassifikator an das Hörgerät, kann der Hörgeräteprüfer (insbesondere manuell) anhand der mittels des Hörgeräts erfassten akustischen Daten überprüfen, ob die von dem Hörgerät ermittelte Hörsituation im vorliegenden Fall plausibel ist (falls beispielsweise die von der Analyseeinheit erfassten akustischen Daten von denen des Hörgeräts abweichen und bereits deshalb unterschiedliche Hörsituationen ermittelt werden).

Das erfindungsgemäße Hörgerätesystem umfasst das Hörgerät, das sowohl durch ein einzelnes Hörgerät als auch durch ein (binaurales) Hörgerätepaar gebildet sein kann, und die dem Hörgerät insbesondere spezifisch zugeordnete (separate) Analyseeinheit. Die Analyseeinheit ist wie vorstehend beschreiben dabei nur zur unidirektionalen Signalübertragung auf das Hörgerät eingerichtet. Das Hörgerät und die Analyseeinheit sind insbesondere softwaretechnisch zur (gemeinsamen) Durchführung des vorstehend beschriebenen Verfahrens zur Ermittlung der trägerspezifischen Nutzungsdaten des Hörgeräts eingerichtet und vorgesehen. Mit anderen Worten ist die Analyseeinheit dazu eingerichtet, ein trägerspezifisches Problem mit dem aktuellen Betriebszustand des Hörgeräts zu ermitteln, dieses als ersten Teildatensatz in einer zugeordneten Speichereinheit zu hinterlegen und daraufhin ein vorzugsweise akustisches Steuersignal an das Hörgerät zu senden. Das Hörgerät umfasst vorzugsweise eine Steuereinheit, die dazu eingerichtet ist, auf den Empfang des Steuersignals hin eine Anzahl von Betriebsdaten des aktuellen Betriebszustands des Hörgeräts zu ermitteln und als zweiten Teildatensatz in einer zugeordneten Speichereinheit zu hinterlegen.

Die erfindungsgemäße Einstelleinheit für das vorstehend beschriebene Hörgerätesystem umfasst jeweils eine (physische oder funkbasierte) Schnittstelle zur (informationsübertragungstechnischen) Kopplung mit der Analyseeinheit und mit dem Hörgerät und ist dazu eingerichtet, das vorstehend beschriebene Verfahren zur Anpassung der Hörgeräteeinstellungen durchzuführen. Die Einstelleinheit kann dabei im Rahmen der Erfindung als nicht-programmierbare elektronische Schaltung ausgebildet sein und hierbei beispielsweise eine speziell zur Anpassung von Hörgeräten ausgebildete Vorrichtung bilden. Vorzugsweise ist die Einstelleinheit aber durch einen Mikrokontroller gebildet, in dem die Funktionalität zur Durchführung des erfindungsgemäßen Verfahrens zur Anpassung der Hörgeräteeinstellungen in Form eines Softwaremoduls implementiert ist. Beispielsweise ist der Mikrokontroller Teil eines Computers, der zur physischen oder drahtlosen Verbindung mit der Analyseeinheit und dem Hörgerät eingerichtet und vorgesehen ist. Die Einstelleinheit ist somit insbesondere dazu eingerichtet, mit dem Hörgerät und der Analyseeinheit informationsübertragungstechnisch verbunden zu werden, anschließend die ersten und zweiten Teildatensätze aus der Analyseeinheit und dem Hörgerät abzurufen (auszulesen), diese miteinander in Verbindung zu bringen und auszuwerten. Zur Auswertung ist die Einstelleinheit vorzugsweise dazu eingerichtet, automatisch anhand der in dem zweiten Teildatensatz enthaltenen Betriebsdaten eine Hörsituation nachzubilden, mit dem ermittelten Problem abzugleichen und anhand dieses Abgleichs eine neue Parametrierung (neue Einstellungen) für das Hörgerät zu ermitteln. Zweckmäßigerweise ist die Einstelleinheit ebenfalls dazu eingerichtet, diese neuen Einstellungen (auf eine Bestätigung insbesondere durch den Hörgeräteakustiker hin) an das Hörgerät zu übertragen.

Herkömmlicherweise bildet die Einstelleinheit einen Teil der hard- und/oder softwaretechnischen Ausstattung eines Hörgeräteakustikers, da für deren Bedienung und insbesondere die Kenntnis der Hörgeräteeinstellungen die besondere Ausbildung und Erfahrung eines Hörgeräteakustikers erforderlich ist. Daher bieten die vorstehend beschriebenen Verfahren und Vorrichtungen den Vorteil, dass der Hörgeräteträger bei Vorliegen eines Problems sich dieses Problem nicht bis zu seinem nächsten Besuch beim Hörgeräteakustiker merken (oder gegebenenfalls aufschreiben) muss. Des Weiteren wird dem Hörgeräteakustiker über die im Hörgerät gespeicherten Betriebsdaten ein hohes Maß an Informationen zur Verfügung gestellt, so dass dieser eine besonders präzise Anpassung der Einstellungen des Hörgeräts vornehmen kann.

Nachfolgend wird ein Ausführungsbeispiel der Erfindung anhand einer Zeichnung näher erläutert. Darin zeigen:
- FIG 1: in schematischer Darstellung ein Hörgerätesystem sowie eine Einstelleinheit für dieses Hörgerätesystem,
- FIG 2: in einem schematischen Ablaufdiagramm ein Verfahren zur Ermittlung von trägerspezifischen Nutzungsdaten des Hörgeräts, und
- FIG 3: in Darstellung gemäß FIG 2 ein Verfahren zur Anpassung von Hörgeräteeinstellungen des Hörgeräts mittels der Einstelleinheit.

Einander entsprechende Teile und Größen sind in allen Figuren stets mit gleichen Bezugszeichen versehen.

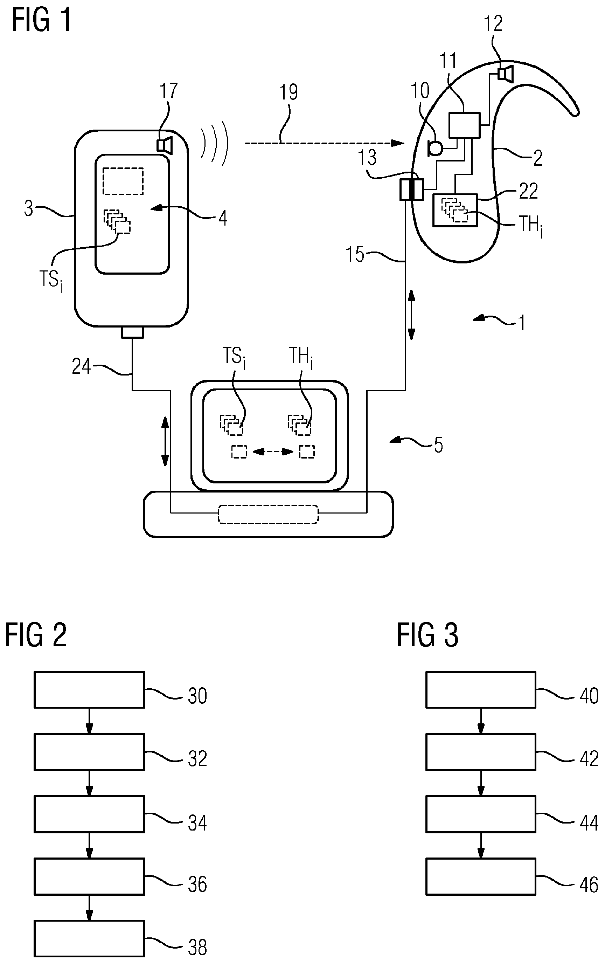

In FIG 1 ist ein Hörgerätesystem 1 dargestellt, das ein Hörgerät 2 und eine als Softwareapplikation auf einem Smartphone 3 lauffähig installierte Analyseeinheit 4 umfasst. Des Weiteren ist in FIG 1 eine Einstelleinheit 5 dargestellt, die als auf einem Rechner (oder Computer) lauffähig installierte Softwareapplikation ausgebildet und dazu eingerichtet ist, zur bidirektionalen Kommunikation mit der Analyseeinheit 4 und dem Hörgerät 2 verbunden zu werden.

Das Hörgerät 2 ist im dargestellten Ausführungsbeispiel als einzelnes sogenanntes Hinter-dem-Ohr-(kurz: HdO-)Hörgerät ausgebildet. Anstelle eines solchen HdO-Hörgeräts kann das Hörgerät 2 im Rahmen der Erfindung jedoch auch als In-dem-Ohr-Hörgerät, als implantierbares Hörgerät oder dergleichen ausgebildet sein. Des Weiteren kann das Hörgerät 2 auch als Hörgerätepaar zur binauralen Versorgung beider Ohren des Hörgeräteträgers ausgebildet sein. Das Hörgerät 2 umfasst zum Empfang von akustischen Signalen ein Mikrofon 10, eine Steuereinheit 11 zur Signalverarbeitung der empfangenen akustischen Signale sowie einen (auch als "Hörer" bezeichneten) Lautsprecher 12 zur akustischen Weitergabe der in der Steuereinheit 11 verarbeiteten (Ton-)Signale an ein Ohr des Hörgeräteträgers. Zur (bidirektionalen) Datenübertragung an die Einstelleinheit 5 umfasst das Hörgerät 2 außerdem eine Datenschnittstelle 13. Die Datenschnittstelle 13 umfasst dabei einen Steckeckanschluss zur Kontaktierung mit einem Datenkabel 15, über das die Kommunikationsverbindung mit (einer korrespondieren Datenschnittstelle) der Einstelleinheit 5 hergestellt wird.

Die Analyseeinheit 4 ist im Gegensatz zur Einstelleinheit 5 nur zur unidirektionalen Kommunikation mit dem Hörgerät 2 ausgebildet. Mit anderen Worten können von der Analyseeinheit 4 nur Daten (insbesondere Befehle) an das Hörgerät 2 übertragen werden, ohne dass ein Informationsfluss in entgegengesetzter Richtung vorhanden oder überhaupt vorgesehen ist. Zur Datenübertragung an das Hörgerät 2 werden hierbei über einen Lautsprecher 17 des Smartphones 3 akustische Signale an das Hörgerät 2, konkret an dessen Mikrofon 10, ausgegeben. Diese akustischen Signale weisen dabei eine im Vergleich zu funkbasierter Datenübertragung nur geringe Übertragungsbandbreite auf, so dass lediglich kurze Datensätze (d. h. mit vergleichsweise kleinem Informationsgehalt) übertagen werden können. Das Smartphone 3 ist durch die installierte Analyseeinheit 4 dazu eingerichtet, in einem nachfolgend näher beschriebenen Verfahren ein (subjektives) Problem des Hörgeräteträgers mit dem aktuellen Betriebszustand des Hörgeräts 2 zu ermitteln und daraufhin über den Lautsprecher 17 des Smartphones 3 ein akustisches Steuersignal 19 an das Hörgerät 2 auszugeben. Das Verfahren dient dazu, (hörgeräte-)trägerspezifische Nutzungsdaten des Hörgeräts 2 zu ermitteln und damit eine (gegenüber einer vorhergehenden Anpassung der Hörgeräteeinstellungen insbesondere durch den Hörgeräteakustiker) nachträgliche Anpassung der Hörgeräteeinstellung zu erleichtern und zu verbessern.

Zunächst wird - wie in FIG 2 dargestellt - mit dem Hörgerätesystem 1 ein Verfahren zur Ermittlung der trägerspezifischen Nutzungsdaten durchgeführt. Dazu wird bei Vorliegen eines Problems des Hörgeräteträgers mit dem Betriebszustand seines Hörgeräts 2 in einem ersten Verfahrensschritt 30 von dem Hörgeräteträger auf seinem Smartphone 3 die Analyseeinheit 4 gestartet. Die Analyseeinheit 4 arbeitet daraufhin in einem zweiten Verfahrensschritt 32 eine Frage-Antwort-Routine ab, indem sie dem Hörgeräteträger eine Anzahl von Fragen, die in einem Fragenkatalog hinterlegt sind, stellt. Die Fragen sind dabei auf bekannte Probleme von Hörgeräteträgern, die mit verschiedenen Betriebszuständen des Hörgeräts in Zusammenhang stehen, abgestimmt. So werden dem Hörgeräteträger zunächst vergleichsweise allgemeine Fragen gestellt, die zur groben Auswahl möglicher Probleme dienen. In Abhängigkeit von der Antwort (insbesondere "richtig" oder "falsch") wird dem Hörgeräteträger anschließend eine (im Vergleich zur vorhergehenden spezifischere) Frage zur Beantwortung vorgelegt. Als beispielhafte Frage-Antwort-Folge ist folgende Reihenfolge denkbar:
- Frage: Betrifft das Problem die eigene Sprache? (mögliche Antwort-Auswahl: richtig/falsch)
- Antwort: richtig.
- Frage: Betrifft das Problem die Lautstärke?
- Antwort: richtig.
- Frage: Ist die eigene Sprache zu laut?
- Antwort: falsch.
- Frage: Ist die eigene Sprache zu leise? Etc.

Am Ende dieser Frage-Antwort-Routine wird dem Hörgeräteträger als Ergebnis das ermittelte Problem (im vorliegenden Beispiel etwa: "Die eigene Sprache wird als zu leise wahrgenommen.") zur Bestätigung vorgelegt. Bestätigt der Hörgeräteträger das ausgewählte/ermittelte Problem nicht positiv (indem er insbesondere "falsch" als Antwort wählt), wird die Frage-Antwort-Routine neu gestartet. Alternativ (oder im Rahmen der Erfindung auch zusätzlich) zum Neustart der Frage-Antwort-Routine wird auf dem Smartphone 3 ein Texteingabefenster geöffnet und dem Hörgeräteträger zur manuellen Eingabe des Problems angeboten, so dass der Hörgeräteträger auch ein von den hinterlegten "Muster-Problemen" abweichendes Problem eingeben kann. Auf eine positive Bestätigung des Problems (bzw. gegebenenfalls auf eine manuelle Eingabe) hin speichert die Analyseeinheit 4 in einem weiteren Verfahrensschritt 34 das ermittelte Problem in einem ersten Teildatensatz TSᵢ in einer der Analyseeinheit 4 zugeordneten Speichereinheit. Dabei vergibt die Analyseeinheit 4 dem ersten Teildatensatz TSᵢ als Marker den Index i, wobei dieser fortlaufende, ganzzahlige Werte i = 0, 1, 2,..., n annehmen kann.

In einem nachfolgenden Verfahrensschritt 36 aktiviert die Analyseeinheit 4 den Lautsprecher 17 des Smartphones 3 und sendet über diesen das Steuersignal 19 an das Mikrofon 10 des Hörgeräts 2. Zur eindeutigen Zuordnung des Steuersignals 19 zu dem Hörgerät 2 wird in dem Steuersignal 19 eine auf das Hörgerät 2 individuell abgestimmte Adresse übermittelt. Ebenfalls wird in dem Steuersignal 19 der für den aktuell gespeicherten ersten Teildatensatz TSᵢ vergebene Index i übermittelt. Auf den Empfang und die Identifikation des Steuersignals 19 anhand der übermittelten Adresse speichert die Steuereinheit 11 in einem Verfahrensschritt 38 eine vorgegebene Auswahl aktueller Betriebsdaten des Hörgeräts 2 als zweiten Teildatensatz THᵢ in einer der Steuereinheit 11 zugeordneten Speichereinheit 22. Die Steuereinheit 11 ordnet dabei dem zweiten Teildatensatz THᵢ den mit dem Steuersignal 19 übermittelten Index i zu.

Die Anzahl und Auswahl der in dem zweiten Betriebsdatensatz THᵢ gespeicherten Betriebsdaten ist dabei fest vorgegeben und gegenüber der in dem Hörgerät 2, konkret in der Steuereinheit 11 vorhandenen Betriebsdaten eingeschränkt. Konkret werden nur solche Betriebsdaten gespeichert, die zur Behebung aller dem Fragenkatalog zugrunde gelegten Probleme des Hörgeräteträgers mit dem Betriebszustand des Hörgeräts 2 erforderlich sind und/oder für eine möglichst präzise Bestimmung der zum Zeitpunkt der Speicherung vorliegenden Hörsituation relevant sind. Dazu werden Betriebsdaten, wie die von dem Mikrofon 10 für unterschiedliche Frequenzen aufgenommene Eingangspegel, das von der Steuereinheit 11 aktuell durchgeführte (Hör-)Programm (z.B. "Fernsehen", "Gespräch mit Umgebungslärm", etc.), die vom Hörgeräteträger aktuell eingestellte Lautstärke und Balance (letzteres im Fall von einem Hörgerätepaar) herangezogen. Im Rahmen des jeweiligen Programms werden auch die jeweiligen Einstellungen (Parameter) bezüglich frequenzabhängiger Verstärkung, Kompression und/oder Dämpfung der Eingangssignale gespeichert. Für die Auswahl des Programms umfasst das Hörgerät 2 (konkret die Steuereinheit 11) einen Klassifikator, der anhand der frequenzabhängigen Eingangspegel des Mikrofons 10 eine Hörsituation bestimmt und in einem Automatik-Modus des Hörgeräts 2 das der Hörsituation entsprechende Programm automatisch einstellt. Die von dem Klassifikator bestimmte Hörsituation ("Klassifikation") wird ebenfalls in dem zweiten Betriebsdatensatz THᵢ gespeichert. Alternativ zu dem Automatik-Modus umfasst die Steuereinheit 11 auch einen Manuell-Modus, in dem der Hörgeräteträger das Programm auch manuell einstellen kann.

Durch die paarweise Vergabe der Indizes i ist es möglich, mehrfach hintereinander Probleme mittels der Analyseeinheit 4 zu ermitteln. Die zugehörigen Teildatensätze TSᵢ und THᵢ werden dabei mit jeweils fortlaufend erhöhtem Index gespeichert. Somit wird eine eindeutige (zeitliche) Zuordnung des jeweiligen ersten und zweiten Teildatensatzes TSᵢ bzw. THᵢ ermöglicht.

In einem (weiterführenden, in Fig. 3 dargestellten) Verfahren zur (nachträglichen) Anpassung der Hörgeräteeinstellungen des Hörgeräts 2 werden in einem Verfahrensschritt 40 die Analyseeinheit 4 bzw. das Smartphone 3 mittels eines Datenkabels 24 und das Hörgerät 2 mittels des Datenkabels 15 an die jeweils korrespondierende Datenschnittstelle der Einstelleinheit 5 angeschlossen. In einem nachfolgenden Verfahrensschritt 42 liest die Einstelleinheit 5 den ersten Teildatensatz TSᵢ (gegebenenfalls alle ersten Teildatensätze TSᵢ) aus der Analyseeinheit 4 und entsprechend den zweiten Teildatensatz THᵢ (bzw. alle zweiten Teildatensätze THᵢ) aus dem Hörgerät 2 aus. Anschließend bringt die Einstelleinheit 5 in einem Verfahrensschritt 44 den jeweiligen - anhand des Index i - identifizierbaren ersten Teildatensatz TSᵢ mit dem zugehörigen zweiten Teildatensatz THᵢ in Verbindung (bildet somit einen Nutzungsdatensatz) und wertet diese aus. Die Auswertung beinhaltet eine Rekonstruktion der zum Zeitpunkt der Speicherung vorliegenden Hörsituation anhand der im zweiten Teildatensatz THᵢ gespeicherten Betriebsdaten des Hörgeräts 2 und ein Abgleichen dieser Hörsituation mit dem zum derzeitigen Zeitpunkt vorliegenden Problem des Hörgeräteträgers. Dadurch ist es auf besonders einfache Weise möglich, die Ursache für das Problem festzustellen und entsprechende Änderungen an den Hörgeräteeinstellungen vorzunehmen.

Die Einstelleinheit 5 ist ferner dazu eingerichtet, in einem Verfahrensschritt 46 anhand des ermittelten Problems und der rekonstruierten Hörsituation eine Anpassung der Hörgeräteeinstellungen zu ermitteln, (dem Hörgeräteakustiker) vorzuschlagen und auf Bestätigung an das Hörgerät 2 zu übertragen. Für den Fall, dass eine Mehrzahl von ersten und zweiten Teildatensätzen TSᵢ bzw. THᵢ hinterlegt ist, werden die Verfahrensschritte 44 und 46 entsprechend mehrfach durchgeführt. Ferner werden von der Einstelleinheit 5 nach dem Auslesen der ersten und zweiten Teildatensätze TSᵢ bzw. THᵢ diese von den jeweiligen Speichereinheiten der Analyseeinheit 4 bzw. des Hörgeräts 2 gelöscht und der jeweilige Index i zurückgesetzt.

In einem weiteren, nicht näher dargestellten Ausführungsbeispiel erfasst die Analyseeinheit 4 im Verfahrensschritt 32 mittels eines Mikrofons des Smartphones 3 zusätzlich die (frequenzabhängigen) Umgebungsgeräusche und hinterlegt diese ebenfalls als Teil des ersten Teildatensatzes TSᵢ. Die Einstelleinheit 5 rekonstruiert dann im Verfahrensschritt 44 anhand dieser - mittels des Smartphones 3 erfassten - Umgebungsgeräusche eine Hörsituation und vergleicht diese mit einer von einem Klassifikator des Hörgeräts 2 (anhand der vom Mikrofon 10 erfassten Tonsignale) ausgegebenen und im zweiten Teildatensatz THᵢ hinterlegten Hörsituation. Dadurch werden eine Verifikation der Funktion des Klassifikators und gegebenenfalls eine Anpassung der Einstellungen des Klassifikators ermöglicht.

In einem ebenfalls nicht dargestellten, alternativen Ausführungsbeispiel ist die Datenschnittstelle 13 des Hörgeräts 2 zur drahtlosen, funkbasierten Kommunikation mit der Einstelleinheit 5 eingerichtet.

Der Gegenstand der Erfindung geht zwar besonders deutlich aus dem vorstehend beschriebenen Ausführungsbeispiel hervor. Dennoch ist der Gegenstand der Erfindung nicht auf das vorstehend beschriebene Ausführungsbeispiel beschränkt. Vielmehr können weitere Ausführungsformen der Erfindung von dem Fachmann aus der vorstehenden Beschreibung abgeleitet werden.

## Patentansprüche

1. Verfahren zur Ermittlung von trägerspezifischen Nutzungsdaten eines Hörgeräts (2), wobei verfahrensgemäß
- mittels einer Analyseeinheit (4), die dem Hörgerät (2) spezifisch zugeordnet, von diesem separat ausgebildet und zur unidirektionalen Signalübertragung auf das Hörgerät (2) eingerichtet ist, ein trägerspezifisches Problem mit dem aktuellen Betriebszustand des Hörgeräts (2) ermittelt wird,
- das ermittelte trägerspezifische Problem in einer der Analyseeinheit (4) zugeordneten Speichereinheit als Teil eines ersten Teildatensatzes (TSᵢ) der Nutzungsdaten hinterlegt wird,
- auf die Ermittlung des trägerspezifischen Problems hin ein Steuersignal (19) an das Hörgerät (2) ausgegeben wird,
- auf den Empfang des Steuersignals (19) hin eine Anzahl von Betriebsdaten des aktuellen Betriebszustands des Hörgeräts (2) ermittelt und als zweiter Teildatensatz (THᵢ) der Nutzungsdaten in einer Speichereinheit (22) des Hörgeräts (2) hinterlegt werden,
wobei der erste Teildatensatz (TSᵢ) und der zweite Teildatensatz (THᵢ) auch nach einer Veränderung des aktuellen Betriebszustands des Hörgeräts (2) in der jeweiligen Speichereinheit (22) hinterlegt bleiben.

2. Verfahren nach Anspruch 1,
wobei das Steuersignal (19) mittels eines der Analyseeinheit (4) zugeordneten Lautsprechers (17) akustisch ausgegeben wird.

3. Verfahren nach Anspruch 1 oder 2,
wobei mittels wenigstens eines der Analyseeinheit (4) zugeordneten Sensors für die aktuelle Situation des Hörgeräteträgers charakteristische Daten ermittelt und in dem ersten Teildatensatz (TSᵢ) der Nutzungsdaten hinterlegt werden.

4. Verfahren nach einem der Ansprüche 1 bis 3,
wobei dem ersten Teildatensatz (TSᵢ) und dem zweiten Teildatensatz (THᵢ) jeweils ein Marker (i) zugewiesen wird, wobei die beiden Marker (i) einer eindeutigen Zuordnung des ersten Teildatensatzes (TSᵢ) und des zweiten Teildatensatzes (THᵢ) zueinander dienen.

5. Verfahren nach Anspruch 4,
wobei von der Analyseeinheit (4) mittels des Steuersignals (19) der Marker (i) für den zweiten Teildatensatz (THᵢ) an das Hörgerät (2) übertragen wird.

6. Verfahren nach einem der Ansprüche 1 bis 5,
wobei zur Ermittlung des trägerspezifischen Problems dem Hörgeräteträger von der Analyseeinheit (4) im Rahmen einer Frage-Antwort-Routine eine Anzahl von Fragen aus einem vorgegebenen, in der Analyseeinheit (4) hinterlegten Fragenkatalog zur Beantwortung vorgelegt wird.

7. Verfahren nach Anspruch 6,
wobei die Frage-Antwort-Routine in der Analyseeinheit (4) auf eine Eingabe des Hörgeräteträgers hin gestartet wird.

8. Verfahren nach einem der Ansprüche 1 bis 7,
wobei für den zweiten Teildatensatz (THᵢ) eine vorgegebene, gegenüber allen im Hörgerät (2) vorhandenen Betriebsdaten eingeschränkte Anzahl der Betriebsdaten herangezogen wird.

9. Verfahren nach einem der Ansprüche 1 bis 7,
wobei in Abhängigkeit von dem ermittelten Problem aus allen im Hörgerät (2) vorhandenen Betriebsdaten eine eingeschränkte Anzahl der Betriebsdaten ausgewählt und für den zweiten Teildatensatz (THᵢ) herangezogen wird.

10. Verfahren zur Anpassung von Hörgeräteeinstellungen eines Hörgeräts (2), wobei verfahrensgemäß
- mittels einer Einstelleinheit (5), die zur bidirektionalen Kommunikation mit dem Hörgerät (2) und mit einer dem Hörgerät (2) zugeordneten Analyseeinheit (4) eingerichtet ist, trägerspezifische Nutzungsdaten des Hörgeräts (2), die gemäß eines Verfahrens nach einem der Ansprüche 1 bis 9 ermittelt wurden, erfasst werden, indem der erste Teildatensatz (TSᵢ) der Nutzungsdaten aus der Analyseeinheit (4) und der zweite Teildatensatz (THᵢ) der Nutzungsdaten aus dem Hörgerät (2) ausgelesen werden,
- der erste Teildatensatz (TSᵢ) mit dem zugehörigen zweiten Teildatensatz (THᵢ) in Verbindung gesetzt wird,
- anschließend beide Teildatensätze (TSᵢ, THᵢ) gemeinsam ausgewertet werden, und
- anhand des Ergebnisses der Auswertung neue Einstellungen für das Hörgerät (2) ermittelt und an das Hörgerät (2) übertragen werden.

11. Hörgerätesystem (1) mit einem Hörgerät (2) und mit einer dem Hörgerät (2) spezifisch zugeordneten, von diesem separat ausgebildeten und zur unidirektionalen Signalübertragung auf das Hörgerät (2) eingerichteten Analyseeinheit (4),
wobei das Hörgerät (2) und die Analyseeinheit (4) zur Durchführung des Verfahrens nach einem der Ansprüche 1 bis 9 eingerichtet sind.

12. Einstelleinheit (5) für ein Hörgerätesystem (1) gemäß Anspruch 10, die jeweils eine Datenschnittstelle zur Kopplung mit der Analyseeinheit (4) und mit dem Hörgerät (2) umfasst, und die dazu eingerichtet ist, das Verfahren gemäß Anspruch 11 durchzuführen.
